(19) Europäisches Patentamt / European Patent Office / Office européen des brevets

(11) **EP 4 556 495 A1**

(12) **EUROPEAN PATENT APPLICATION**
published in accordance with Art. 153(4) EPC

(43) Date of publication:
**21.05.2025 Bulletin 2025/21**

(21) Application number: **23839609.7**

(22) Date of filing: **10.07.2023**

(51) International Patent Classification (IPC):
*C08F 2/44* (2006.01)     *A61K 6/831* (2020.01)
*A61K 6/84* (2020.01)     *A61K 6/887* (2020.01)

(52) Cooperative Patent Classification (CPC):
**A61K 6/831; A61K 6/84; A61K 6/887; C08F 2/44**

(86) International application number:
**PCT/JP2023/025496**

(87) International publication number:
**WO 2024/014437 (18.01.2024 Gazette 2024/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.07.2022 JP 2022111777**

(71) Applicant: **Mitsui Chemicals, Inc.
Tokyo 104-0028 (JP)**

(72) Inventors:
• **ABO, Eri
Sodegaura-shi, Chiba 299-0265 (JP)**
• **TAKAHASHI, Issei
Sodegaura-shi, Chiba 299-0265 (JP)**
• **KOSUGI, Yoko
Sodegaura-shi, Chiba 299-0265 (JP)**

(74) Representative: **Hoffmann Eitle
Patent- und Rechtsanwälte PartmbB
Arabellastraße 30
81925 München (DE)**

(54) **CURABLE COMPOSITION, CURED PRODUCT, AND DENTAL MATERIAL**

(57)     A curable composition includes a monomer, a polymerization initiator, and a filler, and satisfies the following conditions X and Y. In conditions X and Y, log(A) is the common logarithm of viscosity (A), log(B) is the common logarithm of viscosity (B), viscosity (A) is the shear viscosity of the curable composition in mPa·s as measured under measurement conditions of 25°C and a shear rate of 0.50 s$^{-1}$, and viscosity (B) is the shear viscosity of the curable composition in mPa·s as measured under measurement conditions of 25°C and a shear rate of 50.00 s$^{-1}$. Condition X specifies $0.30 \leq \log(A)-\log(B)$, and condition Y provides that viscosity (A) be at least 100,000 mPa·s.

EP 4 556 495 A1

## Description

Technical Field

The present disclosure relates to a curable composition, a cured product, and a dental material.

Background Art

Curable compositions that are called composite resins are used in the dental field, in order to repair chipped teeth. As a curable composition such as a composite resin, for example, Patent Document 1 discloses a dental paste composition that contains two types of filler having different particle sizes.
Patent Document 1: International Publication (WO) No. 2021/132707

SUMMARY OF INVENTION

Problem to be Solved by Invention

There are cases in which it is requested that dripping down of a curable composition at use be reduced. For example, in a case in which a chipping is to be repaired by filling a curable composition for a dental material into the missing portion of a tooth, it may be requested that dripping down of the curable composition filled into the missing portion (for example, flowing out of the missing portion) be reduced.
In order to prevent dripping down of the curable composition, one may think of increasing the viscosity of the curable composition, for example, by increasing the content of a filler or the like.
However, in a case in which the viscosity of the curable composition is increased, extrusion properties during extruding the curable composition from a syringe or the like may deteriorate (i.e., the extruding requires a stronger force).
Therefore, a curable composition may be desired which exhibits reduced tendency to drip down at use and which has excellent extrusion properties despite the lower tendency to drip down.
A problem to be solved by one embodiment of the present disclosure is to provide a curable composition having excellent extrudability and exhibiting reduced tendency to drip down at use, a cured product obtained by curing the curable composition, and a dental material that includes the cured product.

Means for Solving the Problem

Means for solving the problem include the following aspects.

<1> A curable composition, including a monomer, a polymerization initiator, and a filler, wherein the curable composition satisfies the following conditions X and Y:

$$\text{(condition X)} \quad 0.30 \le \log(A)\text{-}\log(B)$$

(condition Y) viscosity (A) is at least 100,000 mPa·s wherein, in conditions X and Y:

log(A) is the common logarithm of viscosity (A),
log(B) is the common logarithm of viscosity (B),
viscosity (A) is the shear viscosity of the curable composition in mPa·s as measured under measurement conditions of 25°C and a shear rate of 0.50 $s^{-1}$, and
viscosity (B) is the shear viscosity of the curable composition in mPa·s as measured under measurement conditions of 25°C and a shear rate of 50.00 $s^{-1}$.

<2> The curable composition according to <1>, wherein the content of the filler is from 40 parts by mass to 2,000 parts by mass with respect to 100 parts by mass of the total amount of the monomer.
<3> The curable composition according to <1> or <2>, wherein the filler includes silicon-containing compound particles.
<4> The curable composition according to <3>, wherein the silicon-containing compound particles have an average primary particle size of from 0.001 μm to 30 μm.
<5> The curable composition according to <3> or <4>, wherein the content of the silicon-containing compound particles is from 40 parts by mass to 2,000 parts by mass with respect to 100 parts by mass of the total amount of the monomer.

<6> The curable composition according to any one of <1> to <5>, wherein the filler includes aluminum oxide particles.

<7> The curable composition according to <6>, wherein the aluminum oxide particles have an average primary particle size of from 1 nm to 500 nm.

<8> The curable composition according to <6> or <7>, wherein the content of the aluminum oxide particles is from 0.1 parts by mass to 25 parts by mass with respect to 100 parts by mass of the total amount of the monomer.

<9> The curable composition according to any one of <1> to <8>, which is a curable composition for a dental material.

<10> A cured product of the curable composition according to any one of <1> to <9>.

<11> A dental material, including at least one of the curable composition according to any one of <1> to <9> or a cured product of the curable composition.

Advantageous Effect of Invention

According to one embodiment of the present disclosure, a curable composition having excellent extrudability and exhibiting reduced tendency to drip down at use, a cured product obtained by curing the curable composition, and a dental material that includes the cured product are provided.

MODES FOR CARRYING OUT INVENTION

In the present disclosure, any numerical range expressed using "to" means a range including numerical values described before and after "to" as a lower limit value and an upper limit value.

In the present disclosure, the term "step" encompasses not only an independent step, but also encompasses a step that cannot be clearly distinguished from other steps, as long as the intended purpose of the step is achieved.

In the present disclosure, in a case in which plural substances corresponding to a component of interest are present in a composition, the amount of the component in the composition means the total amount of the plural substances present in the composition, unless otherwise specified.

For numerical value ranges described in a stepwise manner in the present disclosure, the upper limit value or the lower limit value of one numerical value range may be replaced with the upper limit value or the lower limit value of another numerical value range in the stepwise description. The upper limit value or the lower limit value of any numerical value range described in the present disclosure may also be replaced with a value described in Examples.

In the present disclosure, "light" represents a concept that encompasses active energy radiations such as ultraviolet light and visible light.

In the present disclosure, the term "(meth)acrylate" means acrylate or methacrylate, the term "(meth)acryloyl" means acryloyl or methacryloyl, and the term "(meth)acrylic" means acrylic or methacrylic.

[Curable Composition]

The curable composition according to the present disclosure includes a monomer, a polymerization initiator, and a filler, and satisfies the following conditions X and Y.

$$(\text{condition X}) \quad 0.30 \leq \log(A)\text{-}\log(B)$$

(condition Y) viscosity (A) is at least 100,000 mPa·s

In conditions X and Y, log(A) is the common logarithm of viscosity (A), and log(B) is the common logarithm of viscosity (B). Further, viscosity (A) is the shear viscosity of the curable composition in mPa·s as measured under measurement conditions of 25°C and a shear rate of 0.50 $s^{-1}$, and viscosity (B) is the shear viscosity of the curable composition in mPa·s as measured under measurement conditions of 25°C and a shear rate of 50.00 $s^{-1}$.

Although the curable composition according to the present disclosure exhibits a reduced tendency to drip down at use, the curable composition according to the present disclosure has excellent extrudability (i.e., easy to extrude from a syringe or the like).

Explanation is given below with respect to the above effect.

In the curable composition according to the present disclosure, viscosity (A) is the shear viscosity as measured under measurement conditions of 25°C and a shear rate of 0.50 $s^{-1}$, and correlates with the viscosity of the curable composition at rest.

In the curable composition according to the present disclosure, viscosity (B) is the shear viscosity as measured under measurement conditions of 25°C and a shear rate of 50.00 $s^{-1}$, and correlates with the viscosity of the curable composition at extrusion.

The curable composition according to the present disclosure exhibits a reduced tendency to drip down (hereinafter also expressed as "having excellent anti-dripping property"), due to the curable composition satisfying condition Y (i.e., the condition that viscosity (A), which has a correlation with the viscosity at rest, is at least 100,000 mPa·s).

However, in general, extrudability decreases (i.e., a stronger force is necessary for extrusion) when viscosity (A) of the curable composition is 100,000 mPa·s or more.

In order to address this issue, the curable composition according to the present disclosure satisfies condition X ($0.30 \leq$ log(A)-log(B)), in addition to satisfying condition Y. Due to this configuration, viscosity (B) having a correlation with viscosity at extrusion is maintained low as compared to viscosity (A) having a correlation with viscosity at rest, and, as a result, extrudability is improved.

<Condition X>

Condition X provides that the following inequality be satisfied.

$$0.30 \leq \log(A)\text{-}\log(B)$$

To put it another way, condition X provides that the difference [log(A)-log(B)] be at least 0.30. Extrudability improves due to the difference [log(A)-log(B)] being at least 0.30. From the viewpoint of further improving extrudability, the difference [log(A)-log(B)] is preferably at least 0.50, and more preferably at least 0.80.

The upper limit of the difference [log(A)-log(B)] is not particularly limited. The difference [log(A)-log(B)] is preferably at most 5.00, from the viewpoint of further improving the anti-dripping properties.

Here, log(A) is the common logarithm of viscosity (A), and log(B) is the common logarithm of viscosity (B). Viscosity (A) is the shear viscosity of the curable composition in mPa·s as measured under measurement conditions of 25°C and a shear rate of 0.50 s$^{-1}$, and viscosity (B) is the shear viscosity of the curable composition in mPa·s as measured under measurement conditions of 25°C and a shear rate of 50.00 s$^{-1}$.

A specific example of a measurement method used for measuring shear viscosity (i.e., viscosity (A) and viscosity (B)) is described below.

Shear viscosity (i.e., viscosity (A) and viscosity (B)) can be measured using a rheometer.

The measurement condition to be adopted is, for example, a measurement condition in which 25 cm-diameter parallel plates with a gap of 0.5 mm therebetween are used in the geometry, and in which the temperature is set to 25°C. In the measurement, a dedicated cover (material: polyether ether ketone resin (PEEK), transmittance over the entire wavelength region of from 290 nm to 800 nm is about 0% (at most 0.4%)) capable of covering the measurement area is used for preventing photocuring of the curable composition.

In the measurement of shear viscosity, the shear rate is fixed to 0.0005 s$^{-1}$ during a period in which the time that has elapsed from the start of the measurement (hereinafter referred to as "time t") is from 0 second to 1 second;

the shear rate is increased from 0.0005 s$^{-1}$ to 50.00 s$^{-1}$ during a period in which time t is from 1 second to 2 seconds; and

the shear rate is fixed to 50.00 s$^{-1}$ during a period in which time t is from 2 seconds to 3 seconds.

In the measurement of shear viscosity under the above conditions, shear viscosity at a shear rate of 0.50 s$^{-1}$ is measured as viscosity (A), and shear viscosity at a shear rate of 50.00 s$^{-1}$ is measured as viscosity (B).

<Condition Y>

Condition Y provides that viscosity (A) be at least 100,000 mPa·s. As a result of satisfying this condition, an effect in terms of reducing the dripping down of the curable composition (i.e., an effect in terms of unlikeliness to drip down) can be obtained.

Viscosity (A) is preferably at least 110,000 mPa·s, more preferably at least 300,000 mPa·s, and more preferably at least 500,000 mPa·s, from the viewpoint of further improving the anti-dripping properties.

The upper limit of viscosity (A) is not particularly limited. The upper limit is, for example, 10,000,000 mPa·s.

Components of the curable composition according to the present disclosure are described below.

<Monomer>

The curable composition according to the present disclosure includes at least one monomer (i.e., polymerizable monomer). The curable composition according to the present disclosure may include only one monomer, or may include two or more monomers.

A compound having a radical-polymerizable group is preferably used as a monomer. The radical-polymerizable group is preferably a group containing an ethylenic double bond, and particularly preferably a (meth)acryloyl group.

It is preferable that at least one (meth)acrylate compound is contained as a monomer or monomers. In this case, the proportion of (meth)acrylate compound(s) in the monomer(s) is preferably at least 60% by mass, more preferably at least 80% by mass, and still more preferably at least 90% by mass.

Examples of (meth)acrylate compounds include monofunctional (meth)acrylate compounds and di- or higher-functional (meth)acrylate compounds.

(Meth)acrylate compounds preferably include a di- or higher-functional (meth)acrylate compound, more preferably include a difunctional to hexafunctional (meth)acrylate compound, still more preferably include a difunctional to tetrafunctional (meth)acrylate compound, and particularly preferably include a difunctional (meth)acrylate compound (i.e., di(meth)acrylate compound).

Examples of the difunctional (meth)acrylate compound (i.e., di(meth)acrylate compound) is not particularly limited, and examples thereof include urethane di(meth)acrylate, neopentyl di(meth)acrylate, 1,4-butanediol di(meth)acrylate, 1,6-hexanediol di(meth)acrylate, 1,8-octanediol di(meth)acrylate, 1,9-nonanediol di(meth)acrylate, 1,10-decanediol di(meth)acrylate, tricyclodecane dimethanol di(meth)acrylate, ethyleneglycol di(meth)acrylate, diethyleneglycol di(meth)acrylate, triethyleneglycol di(meth)acrylate, tetraethyleneglycol di(meth)acrylate, polyethyleneglycol di(meth)acrylate, tripropyleneglycol di(meth)acrylate, tetrapropyleneglycol di(meth)acrylate, polypropyleneglycol di(meth)acrylate, 2,2-bis[4-(3-(meth)acryloyloxy-2-hydroxypropoxy)phenyl]propane, ethyleneoxide-modified bisphenol A di(meth)acrylate, and propyleneoxide-modified bisphenol A di(meth)acrylate.

The proportion of di(meth)acrylate compound(s) in the monomer(s) is preferably at least 60% by mass, more preferably at least 80% by mass, and still more preferably at least 90% by mass.

The proportion of di(meth)acrylate compound(s) in the (meth)acrylate compound(s) is preferably at least 60% by mass, more preferably at least 80% by mass, and still more preferably at least 90% by mass.

With respect to monomers, disclosures of known documents, such as JP-A H11-315059, JP-A 2001-70437, JP-A 2011-105722, JP-A 2000-204069, JP-A 2013-544823, WO 2016/125758, WO 2015/119163, and WO 2020/040141, may be referenced, as appropriate.

The content of monomer(s) in the curable composition according to the present disclosure is preferably from 20% by mass to 70% by mass, more preferably from 20% by mass to 65% by mass, and still more preferably from 25% by mass to 60% by mass, with respect to the total amount of the curable composition.

The monomer(s) in the curable composition according to the present disclosure preferably includes a (meth)acrylate compound (A) that contains at least one selected from the group consisting of a urethane bond (*-NH-C(=O)-O-*), a urea bond (*-NH-C(=O)-NH-*), a thiourethane bond (*-NH-C(=O)-S-*), an allophanate bond (*-NH-C(=O)-N(-*)-C(=O)-O-*), an alicyclic structure, and an aromatic cyclic structure, and that has two or more (meth)acryloyl groups. Here, the mark "*" in the bond formulae represents a bonding position.

Including a (meth)acrylate compound (A) in the monomer(s) can further improve the mechanical strength of a cured product of the curable composition according to the present disclosure.

A (meth)acrylate compound (A1) that contains at least one selected from the group consisting of a urethane bond and an aromatic cyclic structure and that has two (meth)acryloyl groups is preferable as the (meth)acrylate compound (A).

When the (meth)acrylate compound (A) includes a urethane bond, the number of urethane bonds is preferably from 2 to 10, and more preferably from 2 to 4.

When the (meth)acrylate compound (A) includes an aromatic cyclic structure, examples of the aromatic cyclic structure include an aryl group (such as a phenyl group) and an arylene group (such as a phenylene group).

When the (meth)acrylate compound (A) includes an aromatic cyclic structure, the number of aromatic rings is preferably from 1 to 20, and more preferably from 2 to 10.

Examples of the (meth)acrylate compound (A1) include 2,2-bis[4-(3-(meth)acryloyloxy-2-hydroxypropoxy)phenyl]propane, ethyleneoxide-modified bisphenol A di(meth)acrylate, propyleneoxide-modified bisphenol A di(meth)acrylate, and bis(2-(meth)acryloylxyethyl) biscarbamate N,N-1,9-nonylene (diurethane (meth)acrylate).

The molecular weight of the (meth)acrylate compound (A1) is preferably from 100 to 5000, more preferably from 200 to 3000, and still more preferably from 300 to 2000. Viscosity (A) can be adjusted to a higher value by setting the molecular weight of the (meth)acrylate compound (A1) to a larger value, and viscosity (A) can be adjusted to a lower value by setting the molecular weight of the (meth)acrylate compound (A1) to a smaller value.

The content of the (meth)acrylate compound (A1) in the curable composition according to the present disclosure is preferably from 4% by mass to 50% by mass, and more preferably from 10% by mass to 36% by mass, with respect to the total mass of the composition.

The curable composition according to the present disclosure preferably includes a (meth)acrylate compound (B), that is different from the (meth)acrylate compound (A).

The (meth)acrylate compound (B) is preferably a (meth)acrylate compound (B1) including at least one selected from the group consisting of: a (meth)acrylate compound (b1) that has two (meth)acryloyl groups but contains none of a urethane bond, a urea bond, a thiourethane bond, an aclicyclic structure, or an aromatic structure; and a (meth)acrylate compound (b2) that has one (meth)acryloyl group.

When the curable composition according to the present disclosure includes a (meth)acrylate compound (B1), which is a monomer having a low viscosity compared to that of a (meth)acrylate compound (A1), viscosity (A) can be adjusted to a lower value.

Examples of the (meth)acrylate compound (b1) include neopentyl di(meth)acrylate, 1,4-butanediol di(meth)acrylate, 1,6-hexanediol di(meth)acrylate, 1,8-octanediol di(meth)acrylate, 1,9-nonanediol di(meth)acrylate, 1,10-decanediol di(meth)acrylate, tricyclodecane dimethanol di(meth)acrylate, ethyleneglycol di(meth)acrylate, diethyleneglycol di(meth)acrylate, triethyleneglycol di(meth)acrylate, tetraethyleneglycol di(meth)acrylate, polyethyleneglycol di(meth)acrylate, tripropyleneglycol di(meth)acrylate, tetrapropyleneglycol di(meth)acrylate, and polypropyleneglycol di(meth)acrylate.

Examples of the (meth)acrylate compound (b2) include cyclohexyl (meth)acrylate, isobornyl (meth)acrylate, dicyclopentanyl (meth)acrylate, dicyclopentenyl (meth)acrylate, 4-tert-butylcyclohexyl (meth)acrylate, tetrahydrofurfuryl (meth)acrylate, (2-methyl-2-ethyl-1,3-dioxolan-4-yl)methyl (meth)acrylate, cyclic trimethylolpropane formal (meth)acrylate, 4-(meth)acryloylmorpholine, lauryl (meth)acrylate, 4-hydroxybutyl (meth)acrylate, 2-hydroxyethyl (meth)acrylate, phenoxyethyleneglycol (meth)acrylate, 2-dodecyl-1-hexadecanyl (meth)acrylate, 2-(meth)acryloyloxyethyl succinate, 2-[[(butylamino)carbonyl]oxy]ethyl (meth)acrylate, and 2-(2-ethoxyethoxy)ethyl (meth)acrylate.

The molecular weight of the (meth)acrylate compound (B1) is preferably from 100 to 500, and more preferably from 150 to 400. Viscosity (A) can be adjusted to a higher value by setting the molecular weight of the (meth)acrylate compound (B 1) to a larger value, and viscosity (A) can be adjusted to a lower value by setting the molecular weight of the (meth)acrylate compound (B 1) to a smaller value.

The content of the (meth)acrylate compound (B1) in the curable composition according to the present disclosure is preferably from 1% by mass to 35% by mass, and more preferably from 2% by mass to 20% by mass, with respect to the total mass of the composition.

When the curable composition according to the present disclosure includes a (meth)acrylate compound (A1) and a (meth)acrylate compound (B1), viscosity (A) can be adjusted to a higher value by adjusting the content of the (meth)acrylate compound (A1) to a larger value, and viscosity (A) can be adjusted to a lower value by adjusting the content of the (meth)acrylate compound (B 1) to a larger value.

The curable composition according to the present disclosure may include a (meth)acrylate compound other than a (meth)acrylate compound (A1) and a (meth)acrylate compound (B1). For example, the curable composition may include a (meth)acrylate compound having a larger molecular weight than that of a (meth)acrylate compound (A1), with a view to further improving viscosity (A).

The total proportion of (meth)acrylate compound (A1) and (meth)acrylate compound (B 1) in (meth)acrylate compound(s) contained in the curable composition according to the present disclosure is preferably at least 60% by mass, more preferably at least 80% by mass, still more preferably at least 90% by mass, and particularly preferably at least 95% by mass.

<polymerization Initiator>

The curable composition according to the present disclosure includes a polymerization initiator. The curable composition according to the present disclosure may include only one polymerization initiator, or may include two or more polymerization initiators.

The polymerization initiator may be a polymerization initiator formed of a combination of two or more compounds.

Examples of the polymerization initiator formed of a combination of two or more compounds include a polymerization initiator formed of a combination of an α-diketone and a reducing agent, a polymerization initiator formed of a combination of a ketal and a reducing agent, and a polymerization initiator formed of a combination of thioxanthone and a reducing agent.

Examples of the α-diketone include camphorquinone, benzil, and 2,3-pentanedione.

Examples of the ketal include benzyl dimethyl ketal and benzyl diethyl ketal.

Examples of the thioxanthone include 2-chlorothioxanthone and 2,4-diethylthioxanthone.

Examples of the reducing agent include tertiary amines such as Michler's ketone (also known as 4,4'-bis(dimethylamino)benzophenone), 2-(dimethylamino)ethyl methacrylate, N,N-bis[(meth)acryloyloxyethyl]-N-methylamine, ethyl N,N-dimethylaminobenzoate, butyl 4-dimethylaminobenzoate, butoxyethyl 4-dimethylaminobenzoate, N-methyldiethanolamine, 4-dimethylaminobenzophenone, N,N-bis(2-hydroxyethyl)-p-toluidine, and dimethylaminophenanthrol; aldehydes such as citronellal, laurylaldehyde, phthaldialdehyde, dimethylaminobenzaldehyde, and terephthalaldehyde; compounds having a thiol group such as 2-mercaptobenzoxazol, decanethiol, 3-mercaptopropyl trimethoxysilane, 4-mercaptoacetophenone, thiosalicylic acid, and thiobenzoic acid.

In regard to the polymerization initiator, disclosures of known documents such as JP-A 11-315059, JP-A 2001-70437, JP-A 2011-105722, JP-A 2000-204069, JP-A 2013-544823, WO 2016/125758, WO 2015/119163, and WO 2020/040141 may be referenced, as appropriate.

The content of the polymerization initiator contained in the curable composition according to the present disclosure is preferably from 0.01% by mass to 10% by mass, more preferably from 0.1% by mass to 5% by mass, and still more preferably from 0.2% by mass to 2% by mass, with respect to the total monomer amount.

The content of the polymerization initiator contained in the curable composition according to the present disclosure is preferably 0.001% by mass to 5% by mass, more preferably from 0.01% by mass to 3% by mass, and still more preferably from 0.1% by mass to 1% by mass, with respect to the total amount of the curable composition.

<Filler>

The curable composition according to the present disclosure includes a filler. The curable composition according to the present disclosure ma include only one filler, or may include two or more fillers. For example, general fillers for use in dental field may be used as fillers.

The content of filler with respect to 100 parts by mass of a total monomer amount is not particularly limited, and is, for example, from 40 parts by mass to 2000 parts by mass.

The content of filler with respect to 100 parts by mass of a total monomer amount is preferably from 40 parts by mass to 1000 parts by mass, more preferably from 50 parts by mass to 500 parts by mass, and still more preferably from 60 parts by mass to 300 parts by mass.

The value of log(A) - log(B) can be adjusted to a smaller value, by adjusting the content of the filler to a higher content. Further, viscosity (A) can be adjusted to a higher value by adjusting the content of the filler to a higher content, and viscosity (A) can be adjusted to a lower value by adjusting the content of the filler to a lower content.

(Silicon-Containing Compound Particles)

The filler preferably includes silicon-containing compound particles. An aspect in which the filler includes silicon-containing compound particles has the advantage that conditions A and B can easily be satisfied.

From the viewpoint of ease of satisfying conditions A and B, the filler preferably includes at least one of silicate salt particles or silicon dioxide particles, and more preferably includes silicate salt particles.

The silicate salt contained in the silicate salt particles may be aluminum barium silicate, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, or strontium calcium fluoroaluminosilicate glass, and is particularly preferably aluminum barium silicate.

Preferable aspects of the silicon-containing compound particles are described below. The preferable aspects below are also preferable aspects of silicate salt particles.

The average primary particle size of the silicon-containing compound particles is preferably from 0.001 $\mu$m to 30 $\mu$m. An average primary particle size within this range makes it easier to satisfy conditions A and B.

From the viewpoint of further facilitating fulfillment of conditions A and B, the average primary particle size of the silicon-containing compound particles is more preferably from 0.01 $\mu$m to 20 $\mu$m, still more preferably from 0.1 $\mu$m to 10 $\mu$m, and still more preferably from 0.2 $\mu$m to 5 $\mu$m.

Viscosity (A) can be adjusted to a higher value by adjusting the average primary particle size of the silicon-containing compound particles to a smaller value, and viscosity (A) can be adjusted to a lower value by adjusting the average primary particle size of the silicon-containing compound particles to a larger value.

In the present disclosure, the average primary particle size of a filler (for example, silicon-containing compound particles) means a number average primary particle size, and means specifically a value as determined in the following manner.

A cured product of a curable composition according to the present disclosure is prepared, and a cross-section of the cured product is obtained by cutting. A transmission electron micrograph (TEM) of the obtained cross-section is taken, particle sizes of randomly selected 100 particles are obtained, equivalent circle diameters thereof are determined, and the arithmetic mean (number average) of the obtained equivalent circle diameters is calculated.

From the viewpoint of facilitating fulfillment of conditions A and B, the content of silicon-containing compound particles with respect to 100 parts by mass of a total monomer amount is preferably 40 parts by mass to 2000 parts by mass, more preferably from 40 parts by mass to 1000 parts by mass, still more preferably from 40 parts by mass to 500 parts by mass, and still more preferably from 40 parts by mass to 300 parts by mass.

Viscosity (A) can be adjusted to a higher value by adjusting the content of silicon-containing compound particles to a larger content. The value of log(A) - log(B) can also be adjusted easily by adjusting the content of silicon-containing compound particles.

The proportion of silicon-containing compound particles in the total filler amount is preferably at least 50% by mass, and more preferably at least 60 % by mass, from the viewpoint of facilitating fulfillment of conditions A and B.

The upper limit of the proportion of silicon-containing compound particles in the total filler amount may be 100% by mass, or may be less than 100% by mass (for example, 99% by mass).

(Aluminum Oxide Particles)

The filler preferably includes aluminum oxide particles (hereinafter also referred to as "alumina particles"). The aspect in which the filler includes aluminum oxide particles has the advantage of facilitating fulfillment of conditions (A) and (B). In particular, viscosity (A) can be adjusted to a higher value by allowing the filler to include aluminum oxide particles, and the aspect in which the filler includes aluminum oxide particles has the advantage of facilitating fulfillment of conditions A and B.

The average primary particle size of aluminum oxide particles is preferably from 1 nm to 500 nm. The average primary particle size of aluminum oxide particles is more preferably from 1 nm to 300 nm, still more preferably from 1 nm to 200 nm, still more preferably from 1 nm to 100 nm, still more preferably from 1 nm to 50 nm, and still more preferably from 2 nm to 30 nm, from the viewpoint of further facilitating fulfillment of conditions A and B.

Viscosity (A) can be adjusted to a higher value by adjusting the average primary particle size of aluminum oxide particles to a smaller value, and viscosity (A) can be adjusted to a lower value by adjusting the average primary particle size of aluminum oxide particles to a larger value.

The value of log(A) - log(B) can be adjusted to a larger value by adjusting the average primary particle size of aluminum oxide particles to a smaller value, and the value of log(A) - log(B) can be adjusted to a smaller value by adjusting the average primary particle size of aluminum oxide particles to a larger value.

The content of aluminum oxide particles with respect to a total monomer amount of 100 parts by mass is preferably from 0.1 parts by mass to 25 parts by mass, more preferably from 1 part by mass to 25 parts by mass, and still more preferably from 2 parts by mass to 25 parts by mass, from the viewpoint of facilitating fulfillment of conditions A and B.

The proportion of aluminum oxide particles in the total filler amount is preferably from 0.1% by mass to 40% by mass, more preferably from 1% by mass to 40% by mass, and still more preferably from 1% by mass to 35% by mass, from the viewpoint of facilitating fulfillment of conditions A and B.

The aluminum oxide particles contained in the curable composition according to the present disclosure may be surface-treated, or may not be surface-treated. The value of log(A) - log(B) can be adjusted to a smaller value by subjecting the aluminum oxide particles to surface treatment (for example, surface treatment with octylsilane).

In regard to the filler, the disclosures of known documents such as JP-A 11-315059, JP-A 2001-70437, JP-A 2011-105722, JP-A 2000-204069, JP-A 2013-544823, WO 2016/125758, WO 2015/119163, and WO 2020/040141, may be referenced, as appropriate.

<Other Components>

The curable composition according to the present disclosure may include components other than the above-described components, in accordance with necessity. Examples of such other components include polymerization inhibitors, coloring agents (for example, pigments or dyes), microbicidal agents, disinfectants, stabilizers, and preservatives. Also with respect to the other components, the disclosures of the aforementioned known documents may be referenced, as appropriate.

<Applications>

The use of the curable composition according to the present disclosure is not particularly limited. The curable composition according to the present disclosure may be used, without particular limitations, in uses in which the foregoing effects in terms of anti-dripping and extrudability are requested.

The curable composition according to the present disclosure is preferably a curable composition for a dental material, from the viewpoint of utilizing the foregoing effects in terms of anti-dripping and extrudability.

When the curable composition according to the present disclosure is a curable composition for a dental material, and the curable composition according to the present disclosure is, for example, filled into a missing portion of a tooth so as to repair the defect, dripping of the curable composition that has been filled into the missing portion (for example, flowing out of the missing portion) can be reduced, and the curable composition also has an excellent extrudability from a syringe (i.e., the force required for extruding can be reduced).

[Cured Product]

The cured product according to the present disclosure is a cured product of the above-described curable composition according to the present disclosure. The cured product according to the present disclosure is produced by curing the above-described curable composition according to the present disclosure.

The curing of the curable composition according to the present disclosure can be carried out by polymerizing a monomer contained in the curable composition according to the present disclosure. The cured product according to the present disclosure can be obtained through the polymerization.

The curing of the curable composition according to the present disclosure is carried out, for example, by irradiating a photocurable composition according to the present disclosure with light (for example, visible light). In this process, the photocurable composition according to the present disclosure after photoirradiation may further be subjected to heat treatment, thereby improving the mechanical properties of the cured product.

[Dental Material]

The dental material according to the present disclosure includes at least one of the above-described curable composition according to the present disclosure or the cured product of the above-described curable composition according to the present disclosure (i.e., the above-described cured product according to the present disclosure).

The dental material according to the present disclosure is, for example, a composite resin for dental restoration, a resin for a denture base, a lining material for a denture base, an impression material, a luting material (resin cement, resin-added glass ionomer cement, or the like), a dental adhesive (adhesive for orthodontics, adhesive for coating on cavity, or the like), a fissure sealant, a resin block for CAD/CAM, a temporary crown, an artificial tooth material, or the like.

The dental material according to the present disclosure is particularly preferably a composite resin for dental restoration. Examples of the composite resin for dental restoration include a composite resin for crown, a composite resin for filling carious cavity, a composite resin for abutment construction, and a composite resin for restoration by filling.

EXAMPLES

Examples of the present disclosure are provided below. However, the present disclosure is not limited to the examples described below.

The meaning of the abbreviations of compounds used in the Examples described below are listed below.

- Monomers -

UDMA: urethane dimethacrylate
2.6E: ethoxylated bisphenol A dimethacrylate
BisGMA: bisphenol A glycerolate dimethacrylate
3G: triethyleneglycol dimethacrylate
NPG: neopentyl dimethacrylate

- Polymerization Initiators -

CQ: camphorquinone
BEDB: 2-butoxyethyl 4-(dimethylamino)benzoate

- Fillers -

GM8235: commercially available dental filler (aluminum barium silicate) with a tradename GM8235 (manufactured by SCHOTT), having a primary particle size of 1.0 $\mu$m
ALUC: commercially available aluminum oxide (alumina) with a tradename AEROXIDE ALUC (manufactured by Aerosil), having a primary particle size of 13 nm
ALUC805: commercially available aluminum oxide (alumina) with a tradename AEROXIDE ALUC 805 (manufactured by Aerosil), having a primary particle size of 13 nm

[Examples 1 to 13 and Comparative Examples 1 to 4]

Among the ingredients indicated in Tables 1 and 2, ingredients (i.e., monomer(s) and polymerization initiators) other than filler(s) were mixed at 50°C to form a homogeneous liquid component, and the liquid component obtained and the filler(s) were kneaded to obtain a pasty curable composition.

<Measurement and Evaluation>

With respect to the curable composition obtained, viscosity (A) and viscosity (B) were each measured as described below. Based on the measurement results of viscosity (A) and viscosity (B), log(A), log(B), and the difference therebetween (log(A) - log(B)) were calculated.

Further, the anti-dripping property and extrudability of the obtained curable composition were evaluated as described below. Viscosity (A), viscosity (B), log(A) - log(B), anti-dripping property, and extrudability are indicated in Tables 1 and 2.

<Measurement of Viscosity (A) and Viscosity (B)>

Shear rate was measured with respect to the obtained curable composition, and viscosity (A) and viscosity (B) were each obtained.

As described above, viscosity (A) is the shear viscosity of the curable composition in mPa·s as measured under measurement conditions of 25°C and a shear rate of 0.50 s$^{-1}$, and

viscosity (B) is the shear viscosity of the curable composition in mPa·s as measured under measurement conditions of 25°C and a shear rate of 50.00 s$^{-1}$.

The measurement of shear viscosity was carried out using a rheometer (HAAKE MARS) manufactured by Thermo Fischer Scientific.

The measurement condition adopted was a measurement condition in which 25 cm-diameter parallel plates with a gap of 0.5 mm therebetween were used in the geometry, and in which the temperature was set to 25°C. In the measurement, a dedicated cover (material: polyether ether ketone resin (PEEK), transmittance over the entire wavelength region of from 290 nm to 800 nm is about 0% (at most 0.4%)) capable of covering the measurement area was used for preventing photocuring of the curable composition.

In the measurement of shear viscosity, the shear rate was fixed to 0.0005 s$^{-1}$ during a period in which the time that had elapsed from the start of the measurement (hereinafter referred to as "time t") was from 0 second to 1 second;

the shear rate was increased from 0.0005 s$^{-1}$ to 50.00 s$^{-1}$ during a period in which time t was from 1 second to 2 seconds; and

the shear rate was fixed to 50.00 s$^{-1}$ during a period in which time t was from 2 seconds to 3 seconds.

In the measurement of shear viscosity under the above conditions, shear viscosity at a shear rate of 0.50 s$^{-1}$ is measured as viscosity (A), and shear viscosity at a shear rate of 50.00 s$^{-1}$ is measured as viscosity (B).

In the Examples, shear viscosity when time t was 1.98 minutes was measured as the shear viscosity at a shear rate of 0.50 s$^{-1}$ (viscosity (A)), and shear viscosity when time t was 2.09 minutes was measured as the shear viscosity at a shear rate of 50.00 s$^{-1}$ (viscosity (B)).

<Calculation of Log(A) - Log(B)>

Based on the measurement results of viscosity (A) and viscosity (B), log(A) and log(B) were calculated, and the difference (log(A) - log(B)) was obtained by subtracting log(B) from log(A).

<Evaluation of Anti-dripping Property>

1.0 mL of the curable composition obtained above was filled into a syringe, and then a needle tip (exclusive for 1 ml, tradename: "Filling Tip", needle type, manufactured by GC) was attached to the tip portion of the syringe. Then, the curable composition in the syringe was extruded in an amount of 0.1 mL from the tip of the needle tip onto a mixing paper pad, by pushing the plunger, thereby dropping the curable composition on the mixing paper pad. Then, the mixing paper pad, having the curable composition (0.1 mL) dropped thereon, was made to stand vertically in a 37°C environment, and to stand still in this state for 2 minutes. After the 2-minutes standing, the length of the region on the mixing paper pad along which the curable composition dripped down was measured, and the anti-dripping property of the curable composition was evaluated according to the following evaluation criterion.

In the following evaluation criterion, the rank at which the anti-dripping property of the curable composition is highest (i.e., most unlikely to drip) is A.

- Evaluation Criterion for Anti-dripping Property -

A: The length of the region on the mixing paper pad along which the curable composition dripped down was greater than or equal to 0 cm and less than or equal to 1.0 cm.
B: The length of the region on the mixing paper pad along which the curable composition dripped down was greater than 1.0 cm and less than or equal to 2.0 cm.
C: The length of the region on the mixing paper pad along which the curable composition dripped down was greater than 2.0 cm.

<Evaluation of Extrudability>

With respect to the curable compositions of the Examples and Comparative Examples that exhibited rank A or B in the evaluation of anti-dripping property, extrudability was evaluated as described below. 1.0 mL of the curable composition was filled into a syringe, and then a needle tip (exclusive for 1 ml, tradename: "Filling Tip", needle type, manufactured by GC) was attached to the tip portion of the syringe.

Thereafter, the syringe was made to stand vertically such that the side to which the needle tip was attached was the bottom side. In this state, the curable composition in the syringe was extruded from the tip of the needle tip using a plunger, during which the force applied for extrusion was measured using a compact tabletop universal testing machine ES-Z (manufactured by Shimadzu Corporation). Specifically, a crosshead to which a jig for a compression strength test was attached was lowered at 10 mm/minute so as to apply a load and press the plunger, thereby extruding the curable composition in the syringe from the tip of the needle tip. The maximum load [N] during extrusion was measured. Based on the maximum load obtained, extrudability was evaluated according to the following criterion.

In the following criterion, rank A is the rank having the best extrudabity.

- Evaluation Criterion for Extrudability -

A: The maximum load during extrusion was greater than or equal to 0 N and less than or equal to 20.0 N.
B: The maximum load during extrusion was greater than 20.0 N and less than or equal to 27.5 N.
C: The maximum load during extrusion was greater than 27.5 N and less than or equal to 70.0 N.
D: The maximum load during extrusion was greater than 70.0 N.

Table 1

| Composition | | | Comparative Example 1 | Comparative Example 2 | Comparative Example 3 | Comparative Example 4 | Example 1 | Example 2 | Example 3 | Example 4 | Example 5 |
|---|---|---|---|---|---|---|---|---|---|---|---|
| Composition | Monomer | UDMA | 70 | 70 | - | - | - | - | - | - | - |
| | | 2.6E | - | - | 70 | 80 | 70 | 70 | 70 | 70 | 70 |
| | | BisGMA | - | - | - | - | - | - | - | - | - |
| | | 3G | 30 | - | - | 20 | - | - | - | - | - |
| | | NPG | - | 30 | 30 | - | 30 | 30 | 30 | 30 | 30 |
| | Polymerization Initiator | CQ | 0.3 | 0.3 | 0.3 | 0.3 | 0.3 | 0.3 | 0.3 | 0.3 | 0.3 |
| | | BEDB | 0.3 | 0.3 | 0.3 | 0.3 | 0.3 | 0.3 | 0.3 | 0.3 | 0.3 |
| | Filler | Aluminum Barium Silicate Particles: GM8235 | 150 | 233 | 150 | 150 | 142.5 | 222 | 222 | 229 | 229 |
| | | Alumina Particles: AluC | - | - | - | 30 | 7.5 | 11 | - | 4 | - |
| | | Alumina Particles: AluC805 | - | - | - | - | - | - | 11 | - | 4 |
| Shear Viscosity | | Viscosity (A) [mPa·s] | 1564 | 23710 | 12350 | 17787453 | 718700 | 4836330 | 2229551 | 1107000 | 789236 |
| | | Viscosity (B) [mPa·s] | 1716 | 29040 | 3802 | 22367800 | 13610 | 55227 | 138869 | 69100 | 87720 |
| | | log(A)-log(B) | -0.04 | -0.09 | 0.51 | -0.10 | 1.72 | 1.94 | 1.20 | 1.20 | 0.95 |
| Anti-dripping Property | | | C | C | C | A | A | A | A | A | A |
| Extrudability | | | | | | D | A | A | A | A | A |

Table 2

| | | | Example 6 | Example 7 | Example 8 | Example 9 | Example 10 | Example 11 | Example 12 | Example 13 |
|---|---|---|---|---|---|---|---|---|---|---|
| Composition | Monomer | UDMA | - | 70 | 70 | 70 | 70 | - | - | - |
| | | 2.6E | 70 | - | - | - | - | 80 | - | - |
| | | BisGMA | - | - | - | - | - | - | 70 | 70 |
| | | 3G | - | 30 | 30 | - | - | - | 30 | 30 |
| | | NPG | 30 | - | - | 30 | 30 | 20 | - | - |
| | Polymerization Initiator | CQ | 0.3 | 0.3 | 0.3 | 0.3 | 0.3 | 0.3 | 0.3 | 0.3 |
| | | BEDB | 0.3 | 0.3 | 0.3 | 0.3 | 0.3 | 0.3 | 0.3 | 0.3 |
| | Filler | Aluminum Barium Silicate Particles: GM8235 | 44 | 142.5 | 142.5 | 229 | 229 | 160 | 160 | 160 |
| | | Alumina Particles: AluC | - | 7.5 | - | 4 | - | 17 | 4.2 | - |
| | | Alumina Particles: AluC805 | 24 | - | 7.5 | - | 4 | - | - | 4.5 |
| Shear Viscosity | | Viscosity (A) [mPa·s] | 1688755 | 535862 | 124852 | 929803 | 1150761 | 718700 | 171500 | 107500 |
| | | Viscosity (B) [mPa·s] | 246803 | 9885 | 14220 | 127045 | 162842 | 13610 | 82760 | 49050 |
| | | log(A)-log(B) | 0.84 | 1.73 | 0.94 | 0.86 | 0.85 | 1.72 | 0.31 | 0.34 |
| Anti-dripping Property | | | A | A | A | A | A | A | A | B |
| Extrudability | | | A | A | A | A | A | A | A | A |

13

As shown in Tables 1 and 2, curable compositions of the Examples, which included a monomer, a polymerization initiator, and a filler, and satisfied condition X, $0.30 \leq \log(A)-\log(B)$, and condition Y, viscosity (A) being at least 100,000 mPa·s, exhibited an excellent anti-dripping property and excellent extrudability.

Compared to the Examples, Comparative Examples 1 to 3, which did not satisfy condition Y, exhibited a lower anti-dripping property (i.e., higher tendency to drip down). In Comparative Example 4, which did not satisfy condition X, extrudability was lower (i.e., more difficult to extrude).

The disclosure of Japanese Patent Application No. 2022-111777, filed July 12, 2022, is incorporated herein by reference in its entirety.

All publications, patent applications, and technical standards mentioned in this specification are herein incorporated by reference to the same extent as if each individual publication, patent application, or technical standard was specifically and individually indicated to be incorporated by reference.

**Claims**

1. A curable composition, comprising a monomer, a polymerization initiator, and a filler, wherein the curable composition satisfies the following conditions X and Y:
   (condition X) $0.30 \leq \log(A)-\log(B)$
   (condition Y) viscosity (A) is at least 100,000 mPa·s wherein, in conditions X and Y:

   $\log(A)$ is a common logarithm of viscosity (A),
   $\log(B)$ is a common logarithm of viscosity (B),
   viscosity (A) is a shear viscosity of the curable composition in mPa·s as measured under measurement conditions of 25°C and a shear rate of 0.50 s$^{-1}$, and
   viscosity (B) is a shear viscosity of the curable composition in mPa·s as measured under measurement conditions of 25°C and a shear rate of 50.00 s$^{-1}$.

2. The curable composition according to claim 1, wherein a content of the filler is from 40 parts by mass to 2,000 parts by mass with respect to 100 parts by mass of a total amount of the monomer.

3. The curable composition according to claim 1, wherein the filler includes silicon-containing compound particles.

4. The curable composition according to claim 3, wherein the silicon-containing compound particles have an average primary particle size of from 0.001 $\mu$m to 30 $\mu$m.

5. The curable composition according to claim 3, wherein a content of the silicon-containing compound particles is from 40 parts by mass to 2,000 parts by mass with respect to 100 parts by mass of a total amount of the monomer.

6. The curable composition according to claim 1, wherein the filler includes aluminum oxide particles.

7. The curable composition according to claim 6, wherein the aluminum oxide particles have an average primary particle size of from 1 nm to 500 nm.

8. The curable composition according to claim 6, wherein a content of the aluminum oxide particles is from 0.1 parts by mass to 25 parts by mass with respect to 100 parts by mass of a total amount of the monomer.

9. The curable composition according to any one of claims 1 to 8, which is a curable composition for a dental material.

10. A cured product of the curable composition according to any one of claims 1 to 8.

11. A dental material, comprising at least one of the curable composition according to any one of claims 1 to 8 or a cured product of the curable composition.

## INTERNATIONAL SEARCH REPORT

| International application No. |
| --- |
| **PCT/JP2023/025496** |

**A. CLASSIFICATION OF SUBJECT MATTER**

***C08F 2/44***(2006.01)i; ***A61K 6/831***(2020.01)i; ***A61K 6/84***(2020.01)i; ***A61K 6/887***(2020.01)i
FI:   C08F2/44 A; A61K6/831; A61K6/84; A61K6/887

According to International Patent Classification (IPC) or to both national classification and IPC

**B. FIELDS SEARCHED**

Minimum documentation searched (classification system followed by classification symbols)

C08F2/44; A61K6/831; A61K6/84; A61K6/887

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2011-184402 A (KURARAY MEDICAL INC) 22 September 2011 (2011-09-22) claims, paragraph [0041], examples | 1-11 |
| X | JP 2013-14556 A (KURARAY NORITAKE DENTAL INC) 24 January 2013 (2013-01-24) claims, examples | 1-11 |
| X | JP 2022-101350 A (KURARAY NORITAKE DENTAL INC) 06 July 2022 (2022-07-06) claims, examples | 1-9, 11 |
| A | | 10 |
| P, X | JP 2022-157070 A (COSMO OIL LUBRICANTS CO LTD) 14 October 2022 (2022-10-14) claims, examples | 1-2, 10 |
| P, A | | 3-9, 11 |
| A | WO 2021/132707 A1 (KURARAY NORITAKE DENTAL INC) 01 July 2021 (2021-07-01) claims, examples | 1-11 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 August 2023** | **05 September 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2023/025496**

### C.    DOCUMENTS CONSIDERED TO BE RELEVANT

| Category* | Citation of document, with indication, where appropriate, of the relevant passages | Relevant to claim No. |
|---|---|---|
| A | US 2018/0290380 A1 (SAINT-GOBAIN CERAMICS & PLASTICS, INC.) 11 October 2018 (2018-10-11)<br>        claims, examples | 1-11 |

Form PCT/ISA/210 (second sheet) (January 2015)

## INTERNATIONAL SEARCH REPORT
### Information on patent family members

International application No.

**PCT/JP2023/025496**

| Patent document cited in search report | | | Publication date (day/month/year) | Patent family member(s) | | | Publication date (day/month/year) |
|---|---|---|---|---|---|---|---|
| JP | 2011-184402 | A | 22 September 2011 | (Family: none) | | | |
| JP | 2013-14556 | A | 24 January 2013 | (Family: none) | | | |
| JP | 2022-101350 | A | 06 July 2022 | (Family: none) | | | |
| JP | 2022-157070 | A | 14 October 2022 | (Family: none) | | | |
| WO | 2021/132707 | A1 | 01 July 2021 | US | 2023/0049373 | A1 | |
| | | | | claims, examples | | | |
| | | | | EP | 4082514 | A1 | |
| US | 2018/0290380 | A1 | 11 October 2018 | WO | 2017/066584 | A1 | |
| | | | | claims, examples | | | |
| | | | | EP | 3362265 | A1 | |
| | | | | EP | 3892449 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021132707 A
- JP H11315059 A
- JP 2001070437 A
- JP 2011105722 A
- JP 2000204069 A
- JP 2013544823 A

- WO 2016125758 A
- WO 2015119163 A
- WO 2020040141 A
- JP 11315059 A
- JP 2022111777 A